Europäisches Patentamt

European Patent Office

Office européen des brevets

(19)

(11) Publication number: **0 130 368**
**A1**

(12) **EUROPEAN PATENT APPLICATION**

(21) Application number: **84106119.5**

(22) Date of filing: **29.05.84**

(51) Int. Cl.⁴: **C 07 C 1/24,** C 07 C 15/02,
C 10 G 3/00

(30) Priority: **31.05.83 US 499462**

(43) Date of publication of application: **09.01.85**
**Bulletin 85/2**

(84) Designated Contracting States: **AT BE CH DE FR GB IT**
**LI LU NL SE**

(71) Applicant: **UNION CARBIDE CORPORATION, Old**
**Ridgebury Road, Danbury Connecticut 06817 (US)**

(72) Inventor: **Rabo, Jule Anthony, 19 Windmill Road, Armonk**
**New York 10504 (US)**
Inventor: **Long, Gary Norman, Birch Hill Road, Putnam**
**Valley New York 10579 (US)**
Inventor: **Yang, Chang-Lee, 71 Sharon Drive, Spring**
**Valley New York 10977 (US)**

(74) Representative: **Eggert, Hans-Gunther, Dr.,**
**Räderscheidtstrasse 1, D-5000 Köln 41 (DE)**

(54) **Conversion of methanol to hydrocarbons.**

(57) Methanol is passed over a steam-stabilized, ultra-hydrophobic zeolite Y catalyst to produce $C_5^+$ hydrocarbons with a significant fraction thereof consisting of $C_{10}^+$ hydrocarbons up to about $C_{22}$ material.

EP 0 130 368 A1

- 1 -

CONVERSION OF METHANOL TO HYDROCARBONS

Field of the Invention

The invention relates to the conversion of methanol to hydrocarbons. More particularly, it relates to such conversion of methanol and like materials to hydrocarbons containing a significant fraction of $C_{10}^{+}$ hydrocarbons.

Description of the Prior Art

Catalytic processes for the conversion of methanol to gasoline boiling range hydrocarbons are well known in the art. Such processes have included those that produce primarily liquid hydrocarbons, the major portion of which materials tend to be $C_6^{+}$ aromatic hydrocarbons. Included among such high boiling aromatics thus produced is $C_{10}$ durene, i.e. (1, 2, 4, 5 - tetramethylbenzene), with only a very small amount of hydrocarbons larger than durene being produced in such processes. The presence of durene is sometimes considered undesirable in gasoline because of its characteristic of crystallizing out at low temperatures. This results in the formation of a solid film in process lines, heat exchanger tubes and the like, as well as in the plugging of lines through which such aromatics are passed.

As a result, efforts have been made to reduce the presence of durene in gasoline boiling range hydrocarbons, and to increase the conversion of methanol and other lower alcohols to lighter high octane gasoline components, such as $C_5$ and $C_6$ olefins and isoparaffins. The Owen et al patent, U.S. 3,969,426, discloses such a process for

D-13545

reducing the presence of durene and increasing the conversion of methanol or lower alcohols to gasoline boiling range components of particularly desired volatility and octane rating for practical use in gasoline products. The patent suggests that a variety of catalyst materials possessing a cracking or acid function, such as zeolite cracking catalysts can be employed in the methanol conversion operations. Preferred catalysts are said to contain a portion of an acidic solid, such as one or more crystalline zeolite compositions, intimately dispersed in a highly porous and relatively catalytically inert matrix material, such as a silica-clay-zirconia matrix. Zeolite compositions said to be suitable for this purpose include synthetic faujasite (X and Y types) and modified faujasites including dealuminized faujasite, rare-earth exchanged faujasites, hydrogen Y-type faujasites, stabilized faujasites, ZSM-4, ZSM-5 and ZSM-11 type crystalline zeolites, mordenite and a variety of other such materials. Some preferred compositions are said to include rare-earth exchanged faujasites, particularly Y type crystalline faujasite, ZSM-5 type crystalline zeolites, mordenite type crystalline zeolites and dealuminized mordenite. A combination of such zeolites may also be employed. As indicated by Owen et al, the zeolites may be steamed or unsteamed.

While such efforts as described by Owen et al are directed toward a reduction in the production of higher boiling hydrocarbons up to durene, it will be appreciated that other efforts are also directed to the conversion of methanol, or of synthesis gas, to high boiling liquid hydrocarbons. For example, it is desirable for practical commercial purposes to produce a broad range of gasoline and diesel fuels from methanol or synthesis gas, and development activity has been carried out, and continues to be carried out, in an effort to accomplish this objective. In this regard, it is well known that synthesis gas, i.e. hydrogen and carbon monoxide, will undergo conversion to hydrocarbons, at from about 300°F to about 850°F, under from about one to one thousand atmospheres pressure, in the presence of a wide variety of transition metal catalysts.

In addition to the activity of a particular catalyst material and its selectivity with respect to the desired conversion product, e.g. gasoline and diesel oil boiling hydrocarbons of present interest, the maintenance of catalytic activity, i.e. catalyst stability, is an important characteristic of a catalyst material for use in a desired catalytic reaction. For example, Y-type zeolite compositions are known to possess a high degree of catalytic activity, but have heretofore been found to lack even a minimal stability when applied in the desired conversion of methanol to hydrocarbons. Thus, Y-82 zeolite is well known for its catalytic activity and is widely used in commercial operations, on a world-wide basis, in the petroleum refining industry, as in hydrocracking to produce gasoline from heavy boiling petroleum feedstocks. When employed as a catalyst for the subject methanol conversion reaction, however, said Y-82 zeolite is found to very rapidly coke up and lose its catalytic activity. Y-82 shows no practical catalytic activity for methanol conversion, therefore, because

D-13545

0130368

- 4 -

of its lack of a requisite minimal degree of catalyst stability for conventional fixed bed processes. As a result, Y-82 and such materials are not useful as catalysts for use in practical commercial fixed bed methanol conversion operations.

In light of such considerations of catalytic activity, selectivity and stability, there remains a desire in the art for a process capable of converting methanol to hydrocarbon products boiling throughout the gasoline and diesel oil range on a practical commercial basis.

It is an object of the invention, therefore, to provide a process for the conversion of methanol and its homologs and ethers to high boiling hydrocarbon products.

It is another object of the invention to provide a process for the production of hydrocarbons boiling throughout the gasoline and diesel oil range from methanol and like materials on a practical commercial basis.

It is a further object of the invention to provide a process capable of converting methanol and like materials to hydrocarbons having an appreciable fraction consisting of materials with more carbon atoms than $C_{10}$ durene boiling in the whole diesel oil range.

With these and other objects in mind, the invention is hereinafter described in detail, the novel features thereof being particularly pointed out in the appended claims.

## Summary of the Invention

Methanol, and its homologs and ethers, are converted to hydrocarbon mixtures consisting of $C_5^+$ materials in the presence of

D-13545

steam-stabilized ultrahydrophobic zeolite Y catalyst. Such mixtures include durene and relatively large amounts of $C_{10}^+$ hydrocarbons up to about $C_{22}$ material.

### Detailed Description of the Invention

The objects of the invention are accomplished by carrying out the desired conversion of methanol and like materials in the presence of specifically modified forms of zeolite Y. More particularly, such forms of zeolite Y are, in contrast to Y zeolite itself, very thermally stable materials that are organophilic, i.e. that exhibit, to a unique degree, an adsorptive preference for less polar organic molecules relative to strongly polar molecules such as water. Such zeolite compositions have been referred to in the art as ultrahydrophobic type Y zeolites, or simply as UHP-Y zeolites. Whereas zeolite Y compositions not modified in the manner of UHP-Y have not exhibited any practical catalytic activity for methanol conversion, the UHP-Y zeolites have been found to possess high activity, selectivity and stability when applied in the process of the invention. In addition to this surprising combination of desirable characteristics, it has also been found, unexpectedly, that the conversion products obtained by the use of such UHP-Y zeolites contain a significant fraction of $C_{10}^+$ hydrocarbons. Very desirably, the hydrocarbon product obtained upon methanol conversion using such UHP-Y zeolites is found to obtain only small amounts of $C_{10}$ durene together with larger amounts of higher molecular weight hydrocarbons up to about $C_{22}$

D-13545

hydrocarbon material. Thus, the process herein described and claimed provides for advantageous means for converting methanol and like materials into a broad range of hydrocarbon products boiling throughout the gasoline and diesel oil range. As the ultrahydrophobic zeolite Y catalyst, i.e. UHP-Y zeolite, is found to possess a suitable catalytic stability for practical commercial operations, the practice of the invention provides a highly desirable process for achieving methanol conversion not limited to a sharp cut off at the upper end of the gasoline boiling range, as occurs with ZSM-5 and other such zeolites, nor resulting in the production of large amounts of distillates heavier than diesel fuel.

The UHP-Y zeolites referred to above are materials known and commercially available in the art, being known and marketed under the designation of LZ-10 by Union Carbide Corporation. The UHP-Y zeolites, prepared by extensive steaming of the low-sodium forms of zeolite Y, are described in Belgium Patent No. 874,373, issued February 22, 1979. As noted therein, such zeolites are organophilic zeolitic aluminosilicate compositions having a $SiO_2$-$Al_2O_3$ molar ratio of from 4.5 to 35, the essential X-ray powder diffraction pattern of zeolite Y, and an ion exchange capacity not greater than 0.070. Furthermore, the zeolites have a crystallographic unit cell dimension, $a_o$, of less than 24.45 Angstroms, a surface area of at least 350 $m^2$/g., a sorptive capacity for water vapor at 25°C and a $p/p_o$ value of 0.10 of less than 5.0 weight percent, and a Residual Butanol Test value of not more than 0.40 weight percent. In preferred compositions, said unit cell dimension of the catalyst is from 24.20 to 24.45 Angstroms. More particularly, the $SiO_2$-$Al_2O_3$ molar ratio for preferred embodiments is from 4.5 to 9.0, and the Residual Butanol Test value is less than 0.35. In addition, the water adsorption capacity at 25°C and a $p/p_o$ value of 0.10 is desirably less than 4.0 weight percent. As referred to above, the surface area of the zeolites is determined by the well-known Brunauer-Emmett-Teller method (B-E-T) (S. Brunauer, P. Emmett and E. Teller, J. Am. Che. Soc. 60, 309 (1938)), using nitrogen as the adsorbate. The essential X-ray powder diffraction pattern of zeolite Y is disclosed in U.S. Patent No. 3,130,007, issued April 21, 1964, said patent being incorporated herein by reference in its entirety in this regard. As indicated in said Belgium patent, the term "ion exchange capacity" is intended to denote the number of active cation sites in the zeolite that exhibit a strong affinity for water molecules and, hence, that appreciably affect the overall capacity of the zeolite to adsorb water vapor. These include all sites that are either occupied by metal or non-metal cations, or which are not occupied by any cation but, in any event, are capable of becoming associated with sodium cations when the zeolite is contacted at 25°C, three times for a period of one hour each, with a fresh aqueous ion exchange solution containing, as the solute, 0.2 mole of NaCl per liter of solution, in proportions such that 100 ml. of solution is used for each gram of zeolite. After such contact of the zeolite with the ion-exchange solution, routine chemical

D-13545

gravimetric analysis is performed to determine the relative molar proportions of $Al_2O_3$, $SiO_2$ and $Na_2O$. Such data are then applied in the formula below to determine the ion exchange capacity (IEC):

$$IEC = k[Na_2O-SiO_2].$$

wherein "k" is the $SiO_2-Al_2O_3$ molar ratio of the zeolite immediately prior to contact with the NaCl ion-exchange solution.

The term "Residual Butanol Test" is a measure of the adsorptive selectivity of zeolite adsorbents for relatively non-polar organic molecules under conditions in which there is active competition between water and less polar molecules for adsorption on the zeolite. The test procedure consists of activating the zeolite sample by heating in air at a temperature of 300°C for 16 hours. The activated zeolite crystals are thereafter slurried with a solution of 1-butanol in water in proportions such that the slurry consists of 1.0 part by weight 1-butanol, 100 parts by weight water and 10 parts by weight of the as-activated zeolite. The slurry is mildly agitated for 16 hours while the temperature is maintained at 25°C. The supernatant liquid is then analyzed for its residual 1-butanol content in terms of weight percent.

For the determination of the sorptive capacity of the UHP-Y zeolite compositions for any particular adsorbate, e.g. water, the test zeolite sample is activated by preheating at 425°C for 16 hours at a pressure of 5 micrometers of mercury in a conventional McBain apparatus. The temperature of the sample is thereafter adjusted to the desired value and contacted with the vapor of the test adsorbate at the desired pressure.

D-13545

The ultrahydrophobic type Y zeolites described above have been found especially suited for use as adsorbents in applications where it is desired to preferentially adsorb organic constituents from solutions or mixtures thereof with water. In the formation of synthesis gas by the distillation of coal, for example, it is desirable, for environmental and economic reasons, to recover the relatively small portion of phenol present in the condensate fraction of principally water that is produced therein. For this purpose, the condensate can be contacted at ambient temperature with a UHP-Y zeolite that will selectively adsorb the phenol from said condensate. UHP-Y zeolites have also been found highly suitable for use as base materials for catalyst compositions having important commercial applications, e.g. in midbarrel hydrocracking catalyst compositions. The catalytic activity exhibited by such UHP-Y zeolites in the practice of the invention is surprising, however, particularly in light of the very low, practically non-existent useful activity exhibited by zeolite Y compositions not modified to the steam-stabilized, ultrahydropholic form referred to above. As will be appreciated by those skilled in the art, zeolite Y compositions, such as Y-82 zeolite, and said modified UHP-Y zeolite compositions possess similarities in crystal structure and chemical composition. Since zeolite Y compositions exhibit a low catalytic activity with respect to the desired methanol conversion, despite its well-recognized and outstanding catalytic activity for other purposes, it would reasonably be foreseen that UHP-Y zeolites would have the same characteristics in light of such similar crystal structure and chemical composition. To the contrary, however, such UHP-Y zeolites have been found uniquely suited for the conversion of methanol and related oxygenated compounds to hydrocarbons, particularly aromatics containing more carbon atoms than $C_{10}$ and up to about $C_{22}$ material, while at the same time exhibiting a practical catalyst life for use in commercial operations.

The benefits obtainable in the practice of the invention are illustrated by the following comparative examples based on the conversion of methanol to $C_5^+$ liquid/solid hydrocarbons. Such examples particularly illustrate the catalytic activity, product selectivity and catalyst stability of four different, representative zeolite and amorphous silica-alumina catalysts under similar reaction conditions. The conversion of methanol in each instance was accomplished in an internal recirculation, continuous-flow stirred Berty reactor having inherent back-mixing of feed and reaction product. Twenty grams of catalyst material were used in each run. Methanol was fed to the reactor at the rate of one weight hourly space velocity, in gm. methanol/gm. catalyst/hr (1 WHSV) in each run. The methanol was employed in a methanol/nitrogen mixture of 0.8/0.2 mole ratio, the nitrogen being introduced with the feed to enable satisfactory reactor pressure control to be maintained at the reaction pressure of 300 psig employed for each run. The methanol was fed during day-time hours only for about 7 hours, with nitrogen flow being maintained continuously overnight. The operation of each run lasted several days and covered a

D-13545

temperature range of 270-380°C. The liquid (with accompanying solid) production was collected, and effluent gas collections were generally made, each day. The liquid product samples contained an aqueous layer, an oil layer and a crystalline hydrocarbon solid at room temperature. These different layers were separated by centifugation and were subjected separately to various analyses. The gas samples were analyzed by gas chromatography. From the results of these analyses, together with knowledge as to the amount of materials fed through the reactor system, the effluent gas flowrate and the weights of liquid and solid products collected, it was possible to reasonably estimate the conversion of methanol and the product selectivity for each run.

The materials employed in the comparative examples represented four different zeolite and amorphous silica-alumina catalyst compositions, namely LZ-105-6 zeolite, Y-82 zeolite, an amorphous silica-alumina gel and LZ-10, a typical, commercially available, ultrahydrophobic, UHP-Y zeolite. It should be noted that LZ-105-6, disclosed in U.S. Patent No. 4,257,895, is a modified crystalline zeolite having similarities in crystal structure and chemical composition to the ZSM-5 type zeolites referred to above. Y-82, as also indicated above, is representative of the zeolite Y catalysts known as possessing outstanding catalytic properties in petroleum refining. In the comparative runs carried out at temperatures above 350°C, LZ-10 was found to exhibit the highest $C_5^+$ hydrocarbon selectivity as is shown in Table I below:

D-13545

- 12 -

## TABLE I

### METHANOL FEED - HYDROCARBON SELECTIVITY

| Catalyst | LZ-105-6 | Y-82 | $SiO_2$-$Al_2O_3$ | LZ-10 |
|---|---|---|---|---|
| Temperature, °C | 369 | 374 | 370 | 371 |
| Hours on Stream | 6 | 7 | 16 | 6 |
| HC Selectivity, in % C-atom | | | | |
| $C_1$ | 0.8 | 29.1 | 18.2 | 13.4 |
| $C_2$ | 1.4 | 2.8 | 1.8 | 2.1 |
| $C_2^=$ | 1.0 | 13.1 | 15.6 | 6.3 |
| $C_3$ | 24.3 | 3.0 | 2.7 | 2.8 |
| $C_3^=$ | 0.4 | 15.3 | 13.9 | 6.4 |
| $C_4$ | 19.7 | 4.0 | 7.8 | 4.0 |
| $C_4^=$ | 0.2 | 8.0 | 8.4 | 2.3 |
| $C_5$ | 7.2 | 3.4 | 5.1 | 1.9 |
| $C_5^=$ | 0.0 | 0.4 | 0.2 | 0.1 |
| $C_6$ | 3.1 | 13.3 | 5.3 | 1.9 |
| $C_7^+$ (in effluent gas) | 1.4 | 6.8 | 13.4 | 1.9 |
| Oil + Solid | 40.5 | 0.8 | 7.6 | 56.9 |
| Total | 100.0 | 100.0 | 100.0 | 100.0 |
| | | | | |
| Total $C_5^+$ in total HC | 52.2 | 24.74 | 31.6 . | 62.7 |
| %420°F$^+$ in total HC | 3.5 | | | 16.8 |
| % 420°F$^+$ in $C_5^+$ HC | 6.7 | | | 26.9 |
| % (420-700°F) in total HC | 3.3 | | | 15.1 |
| % (420-700°F) in $C_5^+$HC | 6.4 | | | 24.1 |

Table I shows the formation of relatively large amounts of hydrocarbons boiling above about 420°F, and particularly in the diesel oil boiling range of from about 420°F to about 700°F, by the use of LZ-10 in the practice of the invention. By contrast and consistent with the prior art discussed above, very little of such hydrocarbons is produced using LZ-105-6 catalyst. Thus, 26.9% of the total $C_5^+$ hydrocarbon product using LZ-10 was 420°F$^+$ material, with 24.1% of said total comprising 420-700°F material, whereas only 6.7% of said 420°F$^+$ material, 6.4% in the 420-700°F range, was produced using LZ-105-6. Those skilled in the art will appreciate that such 420-700°F hydrocarbon material comprises molecules with more carbon atoms than $C_{10}^+$ hydrocarbons up to about $C_{22}$ material. Essentially no $C_{10}^+$ material was produced using Y-82 and $SiO_2$-$Al_2O_3$ catalysts.

An additional benefit of the invention, as illustrated in Table I, resides in the production of a substantial amount of light olefins in the course of conversion of methanol by the use of LZ-10 vis-a-vis the results obtainable using LZ-105-6. Thus, a relatively high $C_2$-$C_4$ olefin fraction is produced using LZ-10, while a very much smaller amount of such olefins is produced using LZ-105-6. Such olefins, and the advantageously higher olefin/paraffin ratio obtained in the $C_2$-$C_4$ range by the use of LZ-10, will be understood to be highly desirable with respect to the value of the gases produced during the methanol conversion operation. While particularly high olefin fractions were also obtained using Y-82 and $SiO_2$-$Al_2O_3$

- 14 -

catalysts, such catalysts are not satisfactory for the desired conversion of methanol to hydrocarbons boiling in the gasoline and diesel oil boiling range for the reasons set forth herein.

Those skilled in the art will appreciate that as with the durene produced in the formation of gasoline by prior art processing, higher boiling aromatic materials capable of condensing out are not desirable components of gasoline and diesel boiling range liquids produced from methanol. The conversion of methanol does, however, result in the formation of such material as will be seen in Table I. The solid-containing products obtained from methanol with LZ-10 catalyst at 371°C and from LZ-105-6 catalyst at 369°C were subject to further characterization as summarized in Table II. The solid-containing products obtained using Y-82 and $SiO_2$-$Al_2O_3$ catalysts were not so characterized because of the relatively small amounts of solids obtained using these catalysts.

## TABLE II

### METHANOL FEED - CHARACTERIZATION OF THE SOLID-CONTAINING PRODUCT

| CATALYST | LZ-10 | LZ-105-6 |
|---|---|---|
| Feed | $CH_3OH+N_2$ | $CH_3OH+N_2$ |
| Pressure, psig. | 310 | 300 |
| Temperature, °C | 371 | 369 |
| Duration, hours | 5.6 | 6.0 |
| Feed, cc $CH_3OH$ | 149 | 163 |
| Theoretical HC Yield, gm | 51.6 | 56.5 |
| Solid-Containing HC | | |
| Product Collected: | | |
| Volume HC's, cc | 12 | 25 |
| Approx. wt., gm. | 8 | 18 |
| % Theoretical Yield | 15.4 | 31.9 |

Solid-Containing HC Product Analysis:

|  | LZ-10 | LZ-105-6 |
|---|---|---|
| | 6% Durene (1,2,4,5-tetramethylbenzene) | 85% Durene |
| | 66% Hexamethylbenzene | |
| | 11% Pentamethylbenzene | 15% unidentified |
| | 11% Decamethylbiphenyl | |
| | 6% unidentified | |

D-13545

As will be seen from this Table II, both LZ-105-6 and LZ-10 catalysts made significant amounts of aromatic hydrocarbons under the indicated reaction conditions. The aromatics produced using LZ-105-6 will be seen to comprise mostly $C_{10}$ durene, (1,2,4,5-tetramethylbenzene), with only a few percent of the solid-containing product materials produced consisting of molecules with more carbon atoms than durene. It should be noted that this result is consistent with the general knowledge of the art as described in the background discussion above. By contrast, the hydrocarbons produced by the use of LZ-10 catalyst include only a relatively minor amount of durene and small hydrocarbons, and, surprisingly, a substantial amount of $C_{10}^{+}$ higher methylated benzenes, e.g. hexamethylbenzene and even substantial amounts of bicyclic hydrocarbons, e.g. decamethylbiphenyl, with carbon numbers up to about $C_{22}$.

It is an added advantage of the invention that the process of the invention can be carried out so as, if desired, to optimize the production of such aromatic hydrocarbons that are not produced using LZ-105-6. For example, if such aromatics, or some of them were desired as chemical intermediates or for use as aromatic hydrocarbons, the invention can be practiced so as to produce a substantial amount thereof, e.g. in excess of 13% of the yield in some cases. As was indicated above, the production of such solid aromatics is not desired when the invention is practiced for the purpose of converting methanol to gasoline and diesel oil products. Under such circumstances, the process of the invention can be carried out so as to reduce the amount of such solids produced as part of the hydrocarbon product. Those skilled in the art will appreciate that various operating conditions can be modified in order to reduce, or alternatively, to increase the amount of such solid aromatics produced in the practice of the invention. For example, the use of lower methanol feed concentrations will generally result in lower amounts of solid material. Likewise, the amount of solid product will generally be reduced by the use of relatively lower contact time of the methanol feed with the catalyst employed. In addition, different reactor configurations will generally result in different amounts of solid production, with a fluid bed reactor tending to result in the production of less solids than when an internal recirculation reactor of the type employed in the comparative runs above is utilized.

The results of comparative runs further established that all four of the catalysts employed were active with respect to the conversion of methanol to dimethylether. Only LZ-105-6 and LZ-10, however, demonstrated activity for the conversion of methanol, and its presumed dimethylether reaction intermediate to hydrocarbons. Moreover, the catalytic activity of LZ-105-6 for hydrocarbon production was found to be somewhat superior to that of LZ-10. Nevertheless at higher temperature levels, for example above about 350°C, LZ-10 is found to rapidly attain relatively high catalytic activity levels with respect to the desired production of hydrocarbons. The Y-82 and the silica-alumina gel catalysts, on the other hand, showed no practical catalytic activity levels for hydrocarbon production from methanol.

The fundamental difference in the hydrocarbon product between LZ-105-6 and LZ-10 has been further demonstrated by boiling temperature curves, not shown herein. The results observed therefrom were consistent with the G.C.-mass spectroscopic analyses of the solid-containing products referred to above. Thus, it was established that the LZ-105-6 product has a substantially sharp cut off at the upper end of the gasoline boiling range as expected. Surprisingly, however, the LZ-10 product boils throughout the gasoline and diesel oil boiling range. A similar boiling curve for amorphous silica-alumina shows no sharp peaks, thereby suggesting, in contrast to LZ-105-6 and LZ-10, the lack of shape selectivity influencing the production of specific hydrocarbon products.

While it is notable that the catalytic properties and the products obtained with LZ-105-6 from methanol are similar to those obtained using ZSM-5 zeolite, in light of the similarities in crystal structure and chemical composition between these two different zeolites, it is surprising that LZ-10 and Y-82 zeolites show fundamental differences in catalytic behavior despite their similarity in crystal structure and chemical compositions. Whereas Y-82 has a low activity for methanol conversion in a fixed bed reactor configuration caused by its rapid coking tendencies, despite being well known for possessing outstanding catalytic

D-13545

activity in catalytic cracking and in hydrocracking processes, LZ-10 has been found to be a uniquely suited catalyst for the conversion of methanol and related oxygenated compounds to hydrocarbons. Not only does it possess a high activity for the conversion of methanol and the like to hydrocarbon products, coupled with the ability to produce aromatics with more carbon atoms than $C_{10}$, but it possesses a reasonable catalyst life, or stability, rendering the process of the invention of practical commercial interest.

A measure of the stability of a catalyst can be obtained from the production rate of oil and solid hydrocarbons in grams per hour as a function of hours in stream. With a methanol feed rate of 1 WHSV and a catalyst charge of 20 grams, the maximum hydrocarbon production rate is about 8.8 gm./hour. LZ-105-6 catalyst, which produces substantially only up to about $C_{10}$ hydrocarbons, has been found to be the most stable catalyst, with oil and solid production of 1.6 gm/hr. at 38 on-stream hours compared to 3.4 gm./hr. at 3 hours of on-stream performance. LZ-10 catalyst was found to exhibit the next best stability, with an oil and solid production rate of 1.2 gm./hr. at 5.9 on-stream hours as compared to 1.7 gm./hr. at 3.4 hours. The amorphous silica-alumina catalyst exhibited no comparable activity for the conversion of methanol to hydrocarbons under like conditions, with its oil and solid production rate being only 0.23 gm./hr. at 4.3 on-stream hours and 0.02 gm./hr. at 15 hours. The Y-82 catalyst was found to be completely deactivated after only a very short

0130368

- 20 -

period of use, with its oil and solid production rate being only 0.002 gm./hr. after 4 on-stream hours.

Catalyst deactivation is always a problem associated with the art of catalysis. Its extent depends upon the nature of the catalyst involved as well as the reaction system in which it is being employed. In the hydrocracking of petroleum, a fresh charge of hydrocracking catalyst will generally last for one or more years when operating under a hydrogen atmosphere with a metallic hydrogenating component incorporated in the catalyst. In the case of cycle oil catalytic cracking, on the other hand, a cracking catalyst would deactivate in a matter of seconds or minutes due to coking. A continual withdrawal of a part of the catalyst charge must thus be made for regeneration purposes, as is practiced in fluidized bed reaction systems. The catalyst life of at least six hours exhibited by LZ-10 in the practice of the invention will thus be seen as constituting a reasonable stability to enable use of the catalyst in the practical commercial applications of the invention as herein disclosed and claimed.

The ultrahydrophobic type zeolites employed in the practice of the invention may be prepared by vigorously steaming low-sodium forms of zeolite Y. For this purpose, a Y-type zeolite having a $SiO_2$-$Al_2O_3$ molar ratio of from 4.5 to 6.0, not greater than 3.3 equivalent percent metal cations and having an adsorptive capacity for water, at 25°C and a $p/p_0$ value of 0.10, of at least 6.0 weight percent and a surface area of at least 350 $m^2/g$.,

D-13545

can advantageously be employed. Such a starting material is calcined in an environment comprising from about 0.2 to 20 atmospheres of steam at a temperature of from 725°C to 870°C for a period of time sufficient to reduce the adsorption capacity for water vapor, at 25°C and a $p/p_o$ value of 0.10, to less than 5.0 weight percent. Preferably, a steam environment of from 0.25 to 1.0 atmospheres is employed. The steam can be used in admixture with gases inert toward the zeolite, such as air, nitrogen, helium, hydrogen and the like, particularly when the steam pressure is less than one atmosphere. Further information relating to the production of such UHP-Y compositions is contained in the above-identified Belgium patent. It will be appreciated that the exact method employed for preparing such UHP-Y zeolites does not form a part of the present invention, as such materials are known and are commercially available, as in the form of the LZ-10 zeolite referred to above.

Those skilled in the art will appreciate that the process of the invention, described above in particular with respect to the comparative examples relating to methanol conversion, can likewise be employed to produce a broad range of hydrocarbon products boiling throughout the gasoline and diesel oil range from other feed materials. It is within the scope of the invention to produce the desired $C_5^+$ hydrocarbon mixture containing a small amount of durene and large amounts of $C_{10}^+$ hydrocarbons up to about $C_{22}$ material from an alcohol having from one to three carbon atoms, or its equivalent ether. While methanol is a particularly desirable feed stream for the process, dimethyl ether, ethanol, diethyl ether, propanol and dipropyl ether can also be employed in the desired production of said $C_5^+$ hydrocarbons.

It will also be understood that various changes and modifications can be made in the details of the process without departing from the scope of the invention as set forth in the appended claims. While the exact proportions of liquid and solid hydrocarbon products boiling throughout the gasoline and diesel oil range may vary depending upon the overall conditions and factors pertinent to a given application, at least about 10%, preferably in excess of 20%, more preferably in excess of 25%, of the $C_5^+$ hydrocarbon product will advantageously comprise hydrocarbons with more carbon atoms than $C_{10}$ durene in contrast to the results obtained using LZ-105-6 catalyst. In this regard, it will be noted that durene was described as being an undesirable product in earlier processes for converting methanol to gasoline boiling range components. In contrast to such prior art circumstances, solid aromatic hydrocarbons can be produced in the practice of the invention, as illustrated in Table II above, with such materials being useful products in embodiments not directed to the production of gasoline and diesel oil boiling range liquid products. This capability provides an additional benefit for the practice of the invention. The process conditions of the invention can thus be adjusted, therefore, so as to either enhance the production of liquid heating, motor and aviation fuels or, if so desired, to maximize the

D-13545

production of specific heavy aromatic materials, such as by the production of substantial amounts of hexamethylbenzene and/or of decamethylbiphenyl, useful as chemical intermediates and for other purposes.

While operating conditions outside such ranges may also be employed, the process of the invention is desirably employed at a temperature of from about 250°C to about 650°C and at a pressure of from about 0 to about 1,000 psig. More preferably, said methanol or feed stream conversion is carried out at a reaction temperature of from about 275°C to about 450°C. The reaction pressure is preferably from about 0 to about 350 psig. Those skilled in the art will appreciate that other operating conditions, such as the residence time of the feed stream in contact with the catalyst, the precise nature of the catalyst employed, and of the feed stream will depend upon the particular requirements, limitations and flexibility that may pertain to a given hydrocarbon production application.

The invention, apart from the particular features of any given application, will be seen as providing a major advance in the art of converting methanol and like materials to valuable hydrocarbon products. Whereas the processes heretofore employed in the art were limited essentially to the production of liquid/solid hydrocarbons up to about $C_{10}$, with durene being a generally undesired by-product thereof, the invention surprisingly enables the conversion of the indicated feed materials to a broad, very desirable range of hydrocarbon products boiling throughout the gasoline

- 24 -

and the whole diesel oil range. In addition, the value of the gas produced is beneficial by the high olefin/paraffin ratio obtained in the $C_2-C_4$ range. It will be appreciated that this additional advantage greatly enhances the overall desirability of the methanol conversion process of the invention as described and claimed herein. The flexibility afforded by the invention with respect to the formation of specific $C_{10}^+$ hydrocarbons containing permethylated aromatics, when desired, represents a further advantage of the invention. Apart from this desirable flexibility, however, it will be appreciated that the invention fulfills a very desirable goal in the art by enabling methanol to be converted to gasoline and diesel oil boiling liquids in a convenient and advantageous manner suitable for practical commercial operations.

D-13545

## CLAIMS

1. A process for the conversion of a feed stream comprising an alcohol having from one to three carbon atoms, or its equivalent ether, to a $C_5^+$ hydrocarbon mixture comprising contacting said feed stream with a steam-stabilized, ultrahydrophobic zeolite Y catalyst, the resulting hydrocarbon product containing more than about 10% of $C_5^+$ hydrocarbon molecules with more carbon atoms than $C_{10}$ durene up to about $C_{22}$ material, said product also containing a substantial amount of olefins as contrasted to paraffins in the $C_2-C_4$ range, whereby said feed stream can advantageously be converted to a broad range of hydrocarbon products boiling throughout the gasoline and diesel oil boiling range.

2. The process of Claim 1 in which said zeolite Y catalyst has a $SiO_2-Al_2O_3$ molar ratio of from 4.5 to 35, the essential X-ray powder diffraction pattern of zeolite Y, an ion exchange capacity not greater than 0.070, a unit cell dimension, $a_o$, of less than 24.45 Angstroms, a surface area of at least $350m^2/g.$, a sorptive capacity for water vapor at 25°C and a $p/p_o$ value of 0.10 of less than 5.0 weight percent, and a Residual Butanol Test value of not more than 0.40 weight percent.

3. The process of Claims 1 or 2 in which said product contains more than about 20% of $C_5^+$ hydrocarbon molecules with more carbon atoms than $C_{10}$ durene.

D-13545

4.   The process of Claim 3 in which more than about 25% of said $C_5^+$ product comprises hydrocarbon molecules with more carbon atoms than $C_{10}$ durene.

5.   The process of Claim 2 in which said unit cell dimension is from 24.20 to 24.45 Angstroms.

6.   The process of Claim 5 in which said $SiO_2$-$Al_2O_3$ molar ratio of the catalyst is from 4.5 to 9.0, and said Residual Butanol Test value is less than 0.35.

7.   The process of Claim 2 in which the water adsorption capacity of the catalyst, at 25°C and a $p/p_0$ of 0.10, is less than 4.0 weight percent.

8.   The process of Claim 1 in which said feed stream comprises methanol.

9.   The process of Claim 1 in which said feed stream comprises dimethyl ether.

10.   The process of Claim 1 in which said feed stream comprises ethanol.

11.   The process of Claim 1 in which said feed stream comprises diethyl ether.

12.   The process of Claim 3 in which said feed stream comprises methanol.

13.   The process of Claim 1 in which said catalytic conversion reaction is carried out at a temperature of from about 250°C to about 650°C.

D-13545

14.   The process of Claim 12 in which said reaction temperature is from about 275°C to about 450°C.

15.   The process of Claim 1 in which said catalytic conversion reaction is carried out at a pressure of from about 0 to about 1,000 psig.

16.   The process of Claim 15 in which said reaction pressure is from about 0 to about 350 psig.

17.   The process of Claim 15 in which said feed stream comprises methanol, the temperature of said reaction being from about 250°C to about 650°C.

18.   The process of Claim 17 in which said reaction temperature is from about 275°C to about 450°C.

19.   The process of Claim 18 in which said reaction pressure is from about 0 to about 350 psig.

20.   The process of Claim 19 in which said zeolite Y catalyst has a $SiO_2$-$Al_2O_3$ molar ratio of from 4.5 to 35, the essential X-ray powder diffraction pattern of zeolite Y, an ion exchange capacity not greater than 0.070, a unit cell dimension, $a_o$, of less than 24.45 Angstroms, a surface area of at least 350 $m^2/g$, a sorptive capacity for water vapor, at 25°C and a $p/p_o$ value of 0.10, of less than 5.0 weight percent, and a Residual Butanol Test value of not more than 0.40 weight percent.

21. The process of Claim 20 in which said unit cell dimension of the catalyst is from 24.20 to 24.45 Angstroms.

22. The process of Claim 1 in which said hydrocabons with more carbon atoms than $C_{10}$ durene comprise substantial amounts of hexamethylbenzene.

23. The process of Claim 1 in which said hydrocarbons with more carbon atoms than $C_{10}$ durene comprise substantial amounts of decamethylbiphenyl.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A,P | EP-A-0 096 996 (MOBIL OIL)<br><br>* Claims 1,2; table 3; page 8, line 27; page 16, lines 18-19 * | 1,8,9, 13 | C 07 C 1/24<br>C 07 C 15/02<br>C 10 G 3/00 |
| X,D | US-A-3 969 426 (H. OWEN et al.)<br>* Claims 1,15; table 6 * | 1,8,13 | |
| A | US-A-4 085 155 (T.E. GOODWIN)<br>* Claim 1; example 1 * | 1,22 | |
| X | US-A-4 347 397 (F.G. DWYER et al.)<br>* Claims 1,12 * | 1,8,13 | |
| A | Comptes Rendus des Séances de l'Académie des Sciences, vol. 288, Série C, Janvier-Février 1979, Paris, pages 245-248 | 1 | **TECHNICAL FIELDS SEARCHED (Int. Cl. 3)**<br><br>C 07 C 1/20<br>C 07 C 1/24<br>C 07 C 15/02<br>C 10 G 3/00 |

The present search report has been drawn up for all claims

| Place of search | Date of completion of the search | Examiner |
|---|---|---|
| BERLIN | 16-08-1984 | KNAACK M |